# EUROPEAN PATENT SPECIFICATION

(11) **EP 4 516 338 B1**
(45) Date of publication and mention of the grant of the patent: **06.08.2025**
(21) Application number: 23194639.3
(22) Date of filing: 31.08.2023
(51) Int. Cl.: A61M 5/142, A61M 5/145, A61M 5/168, A61M 5/172

(54) **DRUG DELIVERY DEVICE**
ARZNEIMITTELABGABEVORRICHTUNG
DISPOSITIF D'ADMINISTRATION DE MÉDICAMENT

(43) Date of publication of application: 05.03.2025
(73) Proprietor: Sensile Medical AG, 4600 Olten (CH)
(72) Inventor: WYSS, Thomas, 4528 Zuchwil (CH); KLUSEWICZ, Pawel, 3015 Bern (CH)
(74) Representative: reuteler & cie SA

(56) References cited:
- US-A1- 2009 123 309
- US-A1- 2016 367 751
- US-A1- 2021 369 957

## Description

### TECHNICAL FIELD

This invention relates to a drug delivery device for subcutaneous administration of a liquid drug. The drug delivery device may be in the form of a patch device.

### DESCRIPTION OF RELATED ART

Drug delivery devices in the form of a patch device for mounting on a patient's skin for subcutaneous delivery of liquid drug are known. It is known to provide drug delivery devices in the form of a patch device with a single use disposable component assembled to a reusable component containing drive and control electronics, or as a single disposable component.

The reliability, safety, compactness and ease of use of drug delivery devices worn by a patient is important. For disposable components, the amount of parts and consequently cost of the disposable device is also an important consideration.

There is need to ensure safety and reliability of mechanisms operated automatically in a drug delivery device, while decreasing costs and increasing the compacity of the medical device.

Safety is one of the most important design considerations in a drug delivery device. It is important to ensure that a drug has been properly and completely administered and that any occlusion or other factors that may affect the specified rate of delivery are reliably monitored. One of the important aspects to monitor is in many cases the amount and rate of delivery of the drug to ensure correct dosage over time.

For devices such as patch pumps that are worn by the patient and possibly activated by the patient outside of a health care facility, the safety and reliability also depend on the simplicity of operation and the robustness of any safety procedures designed to determine malfunctioning.

Many drug delivery devices have pump mechanisms based on linear motor drives that push a plunger in the drug cartridge. These mechanisms are complex and bulky. Moreover, to ensure safe administration valves are required to close the fluid flow path when the drug is not being administered. Such problems can be overcome by providing a drug delivery device with a pump system as described in US 2016/367751, WO 2007074363, WO 2015015379, WO2019228895A1, or WO2020069926A1. In the aforementioned conventional systems, flow rates are adjusted by adjusting the speed of the motor drive. This requires a motor that is adapted to function over a wide range of speeds which results in a certain cost and complexity of the motor itself. In such systems, the volume of pumped liquid per full rotation of the rotor depends on the stroke (the axial displacement) of the rotor whereby certain inaccuracies in stroke measurement may occur due to slight tilting of the rotor. In effect, since the pump engine rotor engages elastic sealing members within the stator, it is possible for the rotor to tilt sightly whereby the tilt angle may be measured incorrectly as an axial displacement. Due to the small amplitudes of axial displacement per rotor cycle there is a need to ensure very accurate stroke measurement, this also serving to determine if the pump is functioning properly or if there is an occlusion or a leakage leading to under or over delivery of the liquid drug.

### SUMMARY OF THE INVENTION

In view of the foregoing, it is an object of the invention to provide a drug delivery device, in particular in the form of a patch device, with a disposable unit, or as an entirely disposable device, for administration of a liquid drug, that is safe, reliable, and compact.

It is advantageous to provide a drug delivery device that is easy to use.

It is advantageous to provide a drug delivery device that is economical to produce.

Objects of the invention have been achieved by providing the drug delivery device according to the independent claim(s). Dependent claims set forth various advantageous embodiments of the invention.

Disclosed herein is a drug delivery device comprising a delivery unit and a drive unit, the delivery unit comprising a drug container and a pumping system having a pump engine (38) with a stator and a pump rotor configured to effect an axial piston stroke as a function of the rotation about an axis of rotation of the pump rotor relative to the stator for pumping liquid out of the drug container, the drive unit comprising a housing, an electronic control system, a pump drive including a motor, a motor output transmission shaft driven in rotation by the motor, a rotor position sensing system, and a coupling slidably coupled to the motor output transmission shaft driven in rotation by the motor output transmission shaft, the coupling having a pump side interface engaged with a drive coupling interface of the pump rotor configured for transmitting torque from the motor to the pump rotor and for effecting an axial displacement following an axial displacement of the pump rotor.

The electronic control system comprises a motor drive circuit connected to the motor of the pump drive and at least one rotary position sensor of the rotor position sensing system for controlling the motor and thereby the pumping of a drug from the drug container with the pumping system, the motor drive circuit including a main controller controlling signals for operation of the motor, and a supervision controller separate from the main controller and also connected to said at least one rotary position sensor for the rotor position sensing system, the supervision controller connected to an interlock circuit configured to transmit electrical power from the power source to the motor, the supervision controller configured to actuate the interlock circuit to interrupt power to the motor if the rotor position measurement signal received from the at least one rotary position sensor does not match a set-point value within a predefined tolerance threshold.

In an advantageous embodiment, the rotor position measurement signal comprises or consists of a number of rotations of the rotor and the predefined tolerance threshold is one or more rotations above or below the set-point value.

In an advantageous embodiment, the main controller comprises or consists of an integrated circuit, and the supervision controller comprises or consists of an integrated circuit, the supervision controller and main controller being supplied with power and sensing signals independently of each other.

In an advantageous embodiment, the main controller and the supervision controller each comprise their own crystal oscillator.

In an advantageous embodiment, the motor drive circuit comprises a delivery control module, the delivery control module receiving an axial position measurement of the pump rotor as a function of the rotational position of the pump rotor, wherein the delivery control module is configured to emit an alarm condition defining an occlusion event of the drug delivery device triggered after an axial position of the rotor is measured as a value above an occlusion position threshold, or after an axial displacement amplitude of the rotor is measured below an occlusion displacement threshold, for at least two revolutions of the pump rotor within a window of at least three to no more than ten revolutions.

In an advantageous embodiment, said window is in a range of 5 to 8 revolutions and said alarm condition defining an occlusion event of the drug delivery device is triggered after an axial position of the rotor is measured as a value above an occlusion position threshold, or after an axial displacement amplitude of the rotor is measured below an occlusion displacement threshold, for at least three revolutions of the pump rotor.

In an advantageous embodiment, the rotor axial position used for determining if the occlusion position threshold has been passed, or the rotor axial displacement used for determining if the occlusion displacement threshold has been passed, is measured within an occlusion window defined by a rotational position of the rotor between an end of expel and a beginning of intake of a pump cycle defined by one revolution of the rotor, the occlusion window being in a both valves closed angular range.

In an advantageous embodiment, the motor drive circuit comprises a delivery control module for controlling operation of the motor of the pump drive, the delivery control module configured to operate the motor in a plurality of pulse and pause sequencies, the pulse and pause times adjusted to achieve a specified average flow rate, each pulse operation comprising a plurality of motor output transmission shaft revolutions, the average flow rate being adjusted by varying the number of revolutions per pulse, or the duration of the pause between pulses.

In an advantageous embodiment, the rotor position sensing system is connected to the electronic control system configured to supply at least a rotor position measurement at least once per revolution to the delivery control module wherein the output transmission shaft is configured to be stopped by the delivery control module in a predefined park position at the end of each pulse operation.

In an advantageous embodiment, the delivery control module is configured to operate pulses having at least three revolutions per pulse, an average flow rate being adjusted by the pause duration alone, or in combination with the pulse duration.

In an advantageous embodiment, the electronic control system is configured to operate a pulse and pause by switching the motor on and off, a voltage and current supply to the motor supplied configured for operation of the motor at a preset optimal power.

In an advantageous embodiment, the rotor position sensing system includes at least one rotary position sensor measuring a rotation of the motor output transmission shaft, and an axial sensor for measuring an axial displacement of the pump rotor, the axial sensor comprising a first axial position sensor mounted statically in the housing of the drive unit, and a second axial position sensor mounted statically in the housing of the drive unit, the first and second axial position sensors arranged on opposite sides of the rotation axis, the first and second angular positions being separated by 180°.

In an advantageous embodiment, the electronic control system is configured to measure an axial position of the coupling side interface by processing a sum or an average value of the measurement outputs of the first and second axial position sensors.

In an advantageous embodiment, the axial position sensor comprises an axial position magnet mounted on the coupling with an annular portion that encircles the rotation axis of the coupling, the first and second axial position sensors comprising first and second magnetic field sensors.

In an advantageous embodiment, the rotary position sensor comprises an integrated incremental solver in the motor.

Further advantageous features of the invention will be apparent from the following detailed description of embodiments of the invention and the accompanying illustrations.

### Brief description of the figures

Figure 1a is a perspective view of a drug delivery device according to an embodiment of the invention;
Figure 1b is a perspective view of a reusable unit and single use disposable unit of the drug delivery device of figure 1a prior to coupling and use;
Figure 1c is a cross-sectional view through the drug delivery device illustrated in figure 1a;
Figure 1d is a cross-sectional view through the drug delivery device illustrated in figure 1a showing the reusable unit and single use disposable unit prior to coupling and use;
Figure 2 is a functional block diagram of a drug delivery device according to an embodiment of the invention;

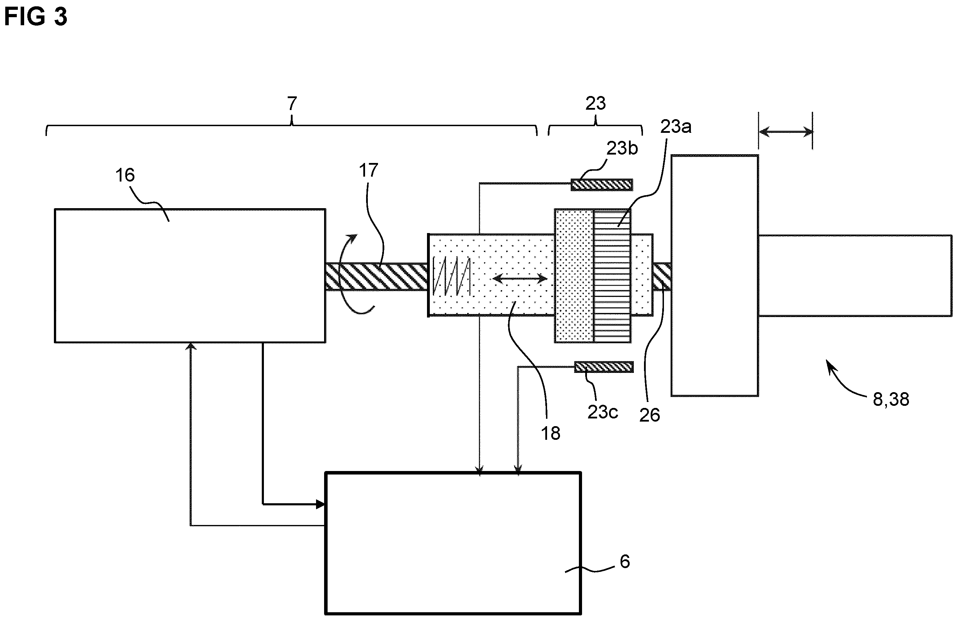

Figure 3 is a simplified schematic illustration of a drug delivery device according to an embodiment of the invention;

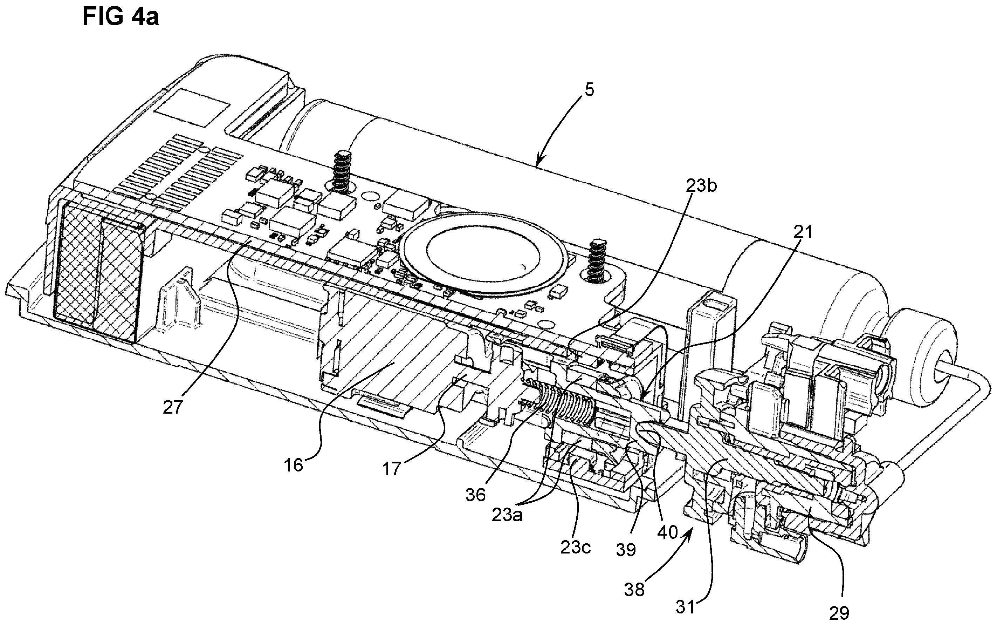

Figure 4a is a perspective cross-sectional view of part of a drug delivery device according to an embodiment of the invention;

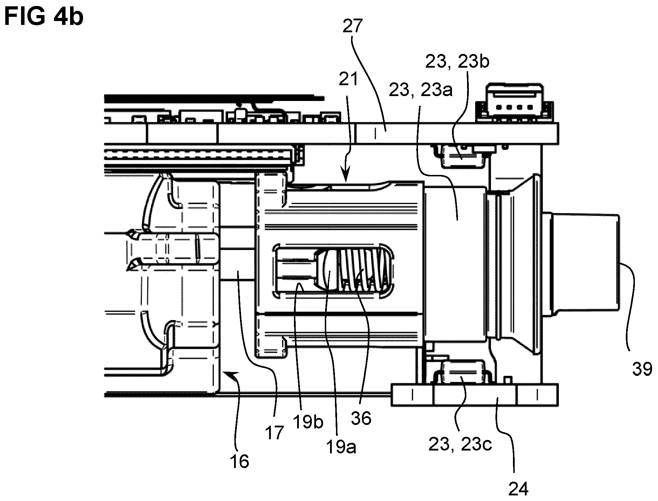

Figure 4b is a side view of a part of the device of figure 4a, showing an output part of a pump drive;

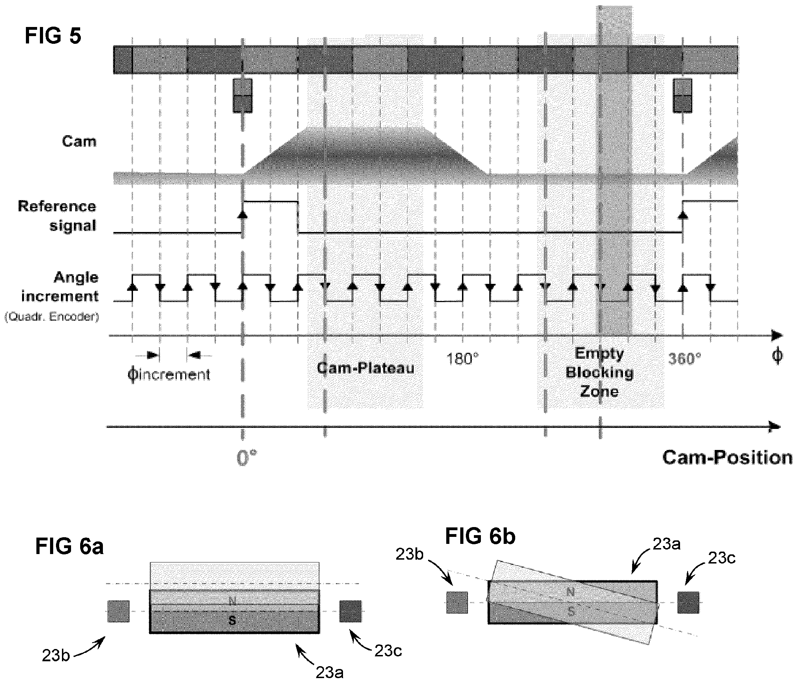

Figure 5 is a diagram illustrating the functioning principle of a rotary and axial displacement of a rotor of the pumping system of a drug delivery device according to embodiments of the invention;
Figures 6a and 6b illustrate schematically axial position sensors (stroke sensors) of a rotor position sensing system of a drug delivery device according to embodiments of the invention;

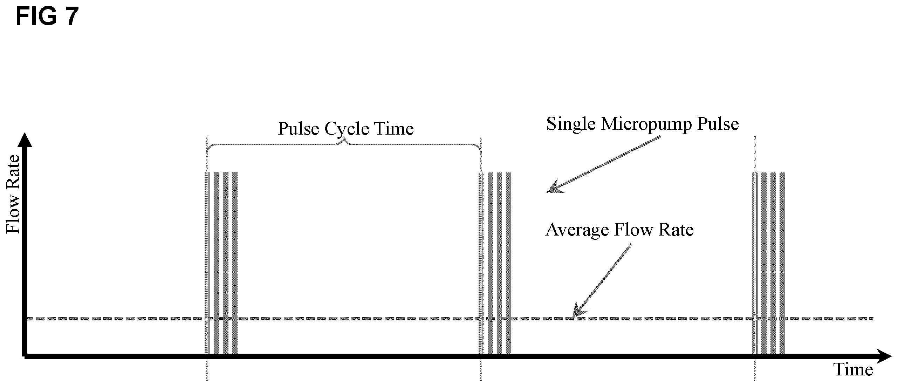

Figure 7 illustrates schematically a plot of flow rate over time of a pump drive and pumping system of a drug delivery device according to an embodiment of the invention;
Figure 8 is a simplified schematic diagram of an electronic control system of a drive unit of a drug delivery device according to an embodiment of the invention;
Figure 9 is a functional block diagram of a reusable unit of a drug delivery device according to an embodiment of the invention;
Figure 10 is a schematic plot of the axial displacement of a rotor as a function of the rotor rotational angle of a pumping system of a drug delivery device according to an embodiment of the invention, the plot illustrating a normal stroke path and an occluded stroke path;
Figure 11 is a graphical illustration of an observation window for determining occlusion based on a certain number of rotor axial position measurements.

### DETAILED DESCRIPTION OF EXEMPLARY EMBODIMENTS

Referring to the figures, a drug delivery device 1 according to embodiments of the invention comprises a housing 2, a delivery unit 3, and a control or drive unit 4, the delivery unit 3 and the control or drive unit 4 being assembled within the housing 2. The housing 2 may be made of two or more parts allowing assembly of the delivery unit, drive unit and any other components within the housing.

In the illustrated embodiments, the drug delivery device for subcutaneous administration of a liquid drug (medicament) comprises a disposable portion formed by the delivery unit that may be assembled to a reusable portion formed by the drive unit, the drive unit including electronics and a power supply. Components of the delivery unit 3 are mounted in a first housing portion of the drug delivery device and components of the drive unit 4 are mounted in a separable second housing portion such that the drive unit 4 can be reused with subsequent delivery units.

Although the embodiments shown in the figures concern a two-part drug delivery device with disposable and reusable units, within the scope of the invention for various aspects described herein, the whole drug delivery device may be provided as a single use disposable unit.

Depending on the medical application, the administration may occur in a single dose over a short period of time, typically less than 1 hour, for instance around 30 minutes or less, or may be for subcutaneous injection of a liquid drug over an extended period of time from a few hours to a few days.

The drug delivery device includes a user interface 46 that may include one or more buttons for actuating the drug delivery device, light and/or sound status indicators, and optionally a screen or other display for presenting information to an operator of the device.

Drug delivery devices according to embodiments of the invention may advantageously be configured as a patch device for mounting on a patient's skin.

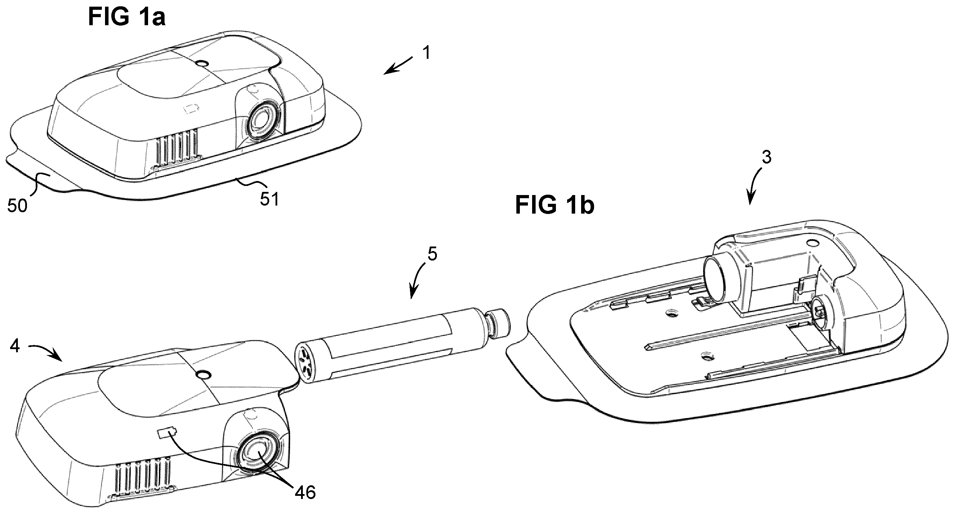

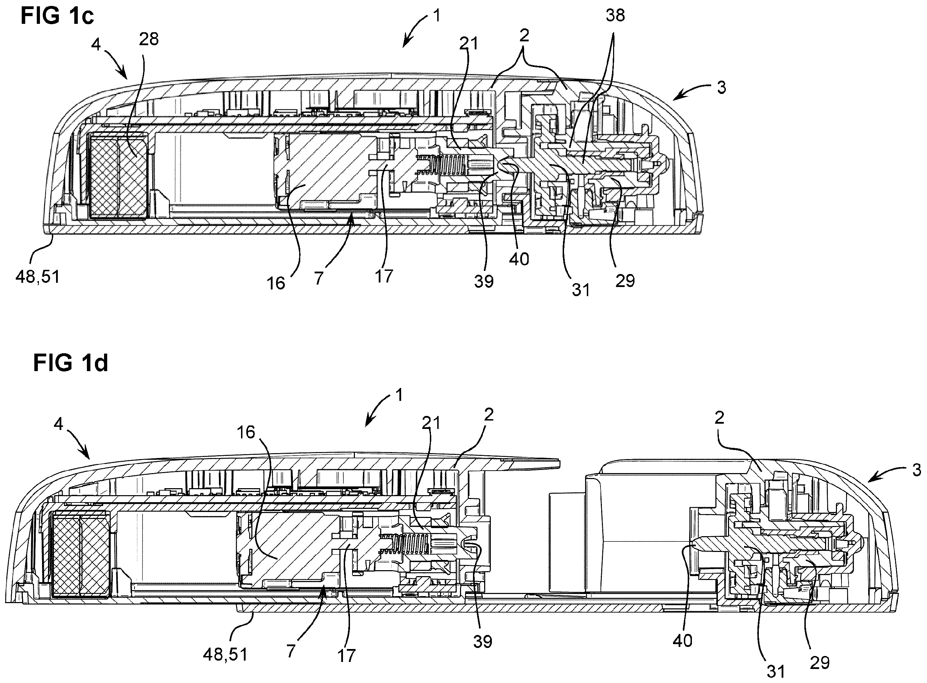

Referring to figures 1a and 1b, in an embodiment, an adhesive layer 51 may be provided on an outer surface of a skin contact wall 48 of the housing 2 covered by a protective film 50 that may be peeled off the adhesive layer 51 prior to placing the adhesive layer on the patient's skin at the site of injection. A needle orifice on the skin contact side is covered by the protective film 50 prior to use, and allows a transcutaneous injection needle (not shown) to extend therethrough and pierce the patient's skin upon activation of the drug delivery device 1.

The delivery unit 3 comprises a drug container 5 containing a liquid drug, for instance a drug cartridge with a stopper as per se well known, and a liquid flow system for channelling the liquid drug to a patient subcutaneously.

The drug delivery device further includes a pumping system that causes liquid from the drug container to be pumped to the injection needle (not shown) once the drug delivery device has been activated.

In an embodiment, the delivery unit 3 incorporates a pump engine 38 of the pumping system 8 that causes liquid from the container to be pumped to the injection needle once the drug delivery device has been activated. The pump engine 38 comprises a drive coupling interface 40 that couples to a complementary coupling interface 39 of a pump drive 7 of the drive unit 4. The drive unit thus provides the mechanical power via the coupling interface 39, 40 to drive the pump engine 38.

The pump engine 38 in the delivery unit 3 may advantageously comprise a design and configuration similar to the pump engine described in WO 2007074363, WO 2015015379, WO2019228895A1, or WO2020069926A1, in which a pump rotor 31 is mounted within a stator 29 and is rotatably and axially movable within the stator 29 in order to pump a fluid from a fluid inlet to a fluid outlet. As known from the above-mentioned publications, the pump rotor 31 has a pump shaft with first and second diameters surrounded by seals that open and close a fluid channel between the inlet and outlet as the rotor rotates and axially displaces due to a cam mechanism between the stator 29 and pump rotor 31, whereby during the opening and closing of the valves between the fluid inlet and pumping chamber, respectively between the pumping chamber and outlet, a pumping action is performed.

An important advantage of using such pump engine in embodiments of the present drug delivery device is that there is no direct connection of fluid between the inlet and outlet at any position of the rotor and no actuation of any valves are required, such that a particularly reliable pumping of liquid without leakage is ensured in an easy to operate arrangement. The pump engine is very compact and can be driven directly by a rotary electrical motor of the pump drive in the drive unit 4 with or without gearing. In effect, because of the differential pumping volume displacement that is defined by the axial displacement of the rotor and the difference between the first and second diameters of the pump shaft, the pumping volume displacement rotation can be easily configured for optimal operation with an electrical motor of a given type rotating with a constant speed. Moreover, the pump engine parts can be made entirely of polymer materials and the rotor may be easily coupled to the pump drive ensuring a sterile barrier between the fluidic portion of the pump engine and the coupling interface.

The drive unit 4 comprises a user interface 46 that may have different configurations depending on the drug to be administered and the intended applications. In some applications the user interface may be very simple having an activation button to start the drug delivery process, and a status indicator for instance a status light and a speaker for example.

Embodiments of the drug delivery device in the form of a patch pump may in particular be for administration of a predetermined amount. Certain applications may simply require administration of a drug in a predetermined dose at a certain time after an event.

One example is for instance the post-operative administration of furosemide after cardiac surgery that requires administration over a period of a few hours a certain amount of time after an operation and at regular intervals, for instance every 24 to 48 hours. The drug delivery device may comprise the drive unit, which forms a re-usable unit, coupled to a disposable unit that includes the delivery unit and cartridge, for adherence to a patient's skin for a single dose delivery over a period of time that may last up to a few hours, after which the drug delivery device is removed and the disposable part thrown away. The re-usable part may be re-used with a subsequent disposable device when the patient next requires an administration of a drug, for instance 48 hours later.

The drive unit 4 further comprises a power source 28 including a battery, an electronic control system 6 and a pump drive 7 that provides the torque driving the pumping system 8 in the disposable delivery unit 3. The drive unit 4 further comprises a rotor position sensing system to monitor and control the pumping operation and to detect under or over delivery, for instance due to occlusion or leakage.

The pump drive 7 comprises a motor 16 having an output transmission shaft 17 that is coupled to a coupling 21 which is axially movable with respect to the output transmission shaft 17 and biased with a spring 36. The coupling 21 has a pump side interface 39 configured to bias against and engage a drive coupling interface 40 of the pump rotor 31 of the pump engine 38 of the delivery unit pumping system. The pump side interface 39 comprises a form complementary to a form of the drive coupling interface 40 of the pump engine 38 configured to transmit torque generated by the motor 16 to the pump engine pump rotor 31. The spring 36 is configured to bias and press the coupling 21 against the pump rotor 31 of the pump engine 38 and to ensure the axial pumping displacement of the rotor in conjunction with cam elements on the pump engine rotor 31 biased against cam elements on the pump engine stator 29. The axial stroke of the coupling 21 of the pump drive 7 thus corresponds to the axial stroke of the pump engine pump rotor 31, and the rotary displacement of the motor output transmission shaft 17 corresponds to the rotary displacement of the pump engine pump rotor 31.

The coupling 21 slidably engages the motor output transmission shaft 17 such that the coupling 21 can move axially, as a function of the rotation of the motor output transmission shaft 17, the axial displacement being defined by the cam arrangement between the pump rotor 31 and stator 29 of the pump engine 38 as *per se* known in the prior art rotary piston pumps as mentioned above.

Although the rotary and axial displacement could be measured on the pump rotor 31 of the pumping system 8 in the delivery unit 3, in order to reduce the number of components, the materials and the complexity of the disposable delivery unit 3, it is more advantageous to include the rotary measurement system and the stroke sensors within the re-usable drive unit 4.

In the illustrated embodiment, the coupling 21 comprises slide grooves 19b on a motor facing side, that are slidably engaged by slide protuberances 19a on a coupling side interface 18 of the motor output transmission shaft 17. It may however be noted that the grooves and protuberances on the coupling and transmission shaft may be inverted, or other mechanical configurations that allow to transmit torque yet allow relative axial movement, such as a spline shaft, may be employed instead of the illustrated embodiments. A plurality of protuberances 19a and corresponding grooves 19b, for instance two, three or four may be distributed around the circumference for better guidance and positioning of the coupling and to reduce the frictional resistance between each protuberance and corresponding sliding engaging surface of the groove.

The rotor position sensing system 9 comprises a rotary position measurement system and an axial position sensor (stroke sensor) 23. The rotor position sensing system 9 is configured to measure the axial and rotational position of the pump engine pump rotor 31 and therefrom also the displacement of the rotor.

The rotary position measurement system may be directly integrated in the motor 16 as per se known in conventional motors, for instance in the form of an increment solver integrated in the motor. Other rotary measurement systems *per se* known for electrical motor drives may be used within the scope of the invention.

In an advantageous embodiment, the rotor position sensing system may further comprise a rotational index sensor 22 that defines at least one rotational position, for instance by means of a discrete magnet or a pair of discrete magnets positioned on the circumference of the motor output transmission shaft 17 to detect a specific rotational position of the output transmission shaft 17. The discrete reference position magnet may for instance be positioned and a corresponding magnetic field sensor positioned on the circuit board 27 or in a housing portion and connected to the circuit board such that a specific rotor position can be detected. The rotational index sensor 22 can also be used to set a reference rotational position for resetting the counting performed by the rotary position sensor 20 to determine an angular position relative to the reference position defined by the rotation index sensor 22. In an embodiment, the rotational index sensor 22 may also be integrated within the motor 16.

The axial position sensor 23 comprises an axial position magnet 23a mounted on the coupling 21, a first axial position sensor 23b for instance in the form of a magnetic field sensor such as a Hall sensor, and a second axial position sensor 23c similar to the first axial position sensor 23b positioned on an opposed side of the coupling, facing the first axial position sensor. The axial position magnet 23a has a north-south magnetic pole orientation that is axially aligned and the displacement of the annular axial position magnet 23a in the axial direction changes the direction and intensity of the magnetic field measured by the first and second axial position sensors 23b, 23c as a function of the axial position relative to the stator.

If there is some tilt in the pump rotor 31 of the delivery unit pumping system 8, due to the biasing of the coupling 21 thereagainst which also follows the tilt of the pump rotor 31 as well as the rotation position, the first and second axial position sensors 23b, 23c will measure a different axial position. The axial position measurement of the first and second axial position sensors however may be summed or averaged in order to determine the true axial position at the center line of the pump rotor 31 that defines the actual or true stroke of the rotor and thus of the pumped volume. Summing of the outputs of the first and second axial position sensor allows to amplify the output of the axial measurement signal.

In addition to providing a more accurate measurement of the pump rotor stroke, the second axial position sensor 23c also provides an additional stroke measurement value to that of the first axial position sensor 23b, which may provide a redundant safety measurement of the axial displacement and thus of the pumped volume of drug. A faulty stroke measurement can thus be more safely detected with the two axial position sensors.

In an advantageous embodiment, the first axial position sensor 23b, which may for instance be in the form a Hall sensor provided in an integrated circuit chip, may advantageously be mounted on the main circuit board 27, and the second axial position sensor 23c may be mounted on a secondary circuit board 24 that is connected via conducting wires or cable to the main circuit board and held to a housing part or relative to the main circuit board with a holder bracket.

The volume of liquid pumped for one pump cycle, namely a 360° rotation of the pump rotor 31, is a fixed volume defined by the axial stroke of the rotor. In conventional pumps, the flow rate of the liquid is varied by varying the rotational speed of the rotor which thus changes the frequency of the axial strokes and thus of the flow rate. This however requires that the motor of the pump drive can supply the required power for pumping over a large range of rotational speeds. However, since an electrical motor typically has a small rotational speed range at which it delivers maximum power, a motor that is used over a large speed range would need to be larger and require more complex control systems than if it were to operate only at a constant speed corresponding to its optimal power to size ratio.

In view of the foregoing, according to an aspect of the invention, the pump drive is configured to operate the motor at a predefined rotational speed, corresponding to the motor's optimal operating point in terms of torque and speed, and adjust the flow rate by switching the motor off for pauses between pulses of the predefined rotational speed. It may however be noted that there may be more than one predefined rotational speed, in other words the motor may also have two optimal operating points and function at two different speeds for adjusting very large operating ranges with pauses and pulses.

During a priming operation when the drug delivery device is started (initial use), or when a bolus administration with the highest flow rate is required, the pumping operation may comprise no pauses and simply rotate at the predefined rotational speed without pauses. The pulses may be of a substantially constant duration, for instance one to ten rotations of the rotor followed by a pause duration that defines the flow rate. Alternatively, the pulse and pause durations may be each adjusted to have each variable duration. It may be noted that the notion of predefined rotational speed comprises a constant nominal speed at steady state operation, it being understood that the motor will of course need to accelerate to the nominal operating speed and then decelerate when switched off. The flow rate command of the motor however depends on the switching on and off times that define the pulse and pause durations, the power supply to the motor phases being the same for each pulse cycle.

It may be noted that the rotor position sensing system 9 may be used to ensure that the rotor is stopped at each end of the pulse duration in a specific park position and subsequently restarted at the next pulse at the same park position in order to ensure that each pulse duration has a specific defined number of full (360°) rotor rotations.

Depending on the required flow rate, different pulse (i.e. switching on and then off) patterns may be implemented.

The pattern of pause and pulses as a function of the flow rate may be stored in a memory 14 of the electronic control system, for instance in the form of a look-up table accessed by the control system for implementing a specified flow rate, or may be computed based on a stored function. In certain medical applications, the flow rate and overall pattern of flow of drug to be administered may be predetermined and factory set in the memory of the drug delivery device.

One example of an application of the drug delivery device for administration of a drug over five hours, with a priming phase upon starting the drug delivery device, followed by a two stage delivery with a higher infusion rate over the first hour and a lower infusion rate over the next four hours is illustrated in the table below.

| **Phase** | **Delivery Pattern** | **Target Volume** | **Duration** | **Pump rotations per Pulse** | **Number of Pulse Cycles** | **Pulse Cycle Time (pulse and pause)** |
|---|---|---|---|---|---|---|
| **1** | On-body priming | n/a | 30 s | 30 | n/a | 120 ± 1 s |
| | 1 | 1 ml ± 10% | 1 h | 17 | 30 | |
| **2** | 2 | 1.67 ml ± 10% | 4 h | 7 | 124 | 116 ± 1 s |

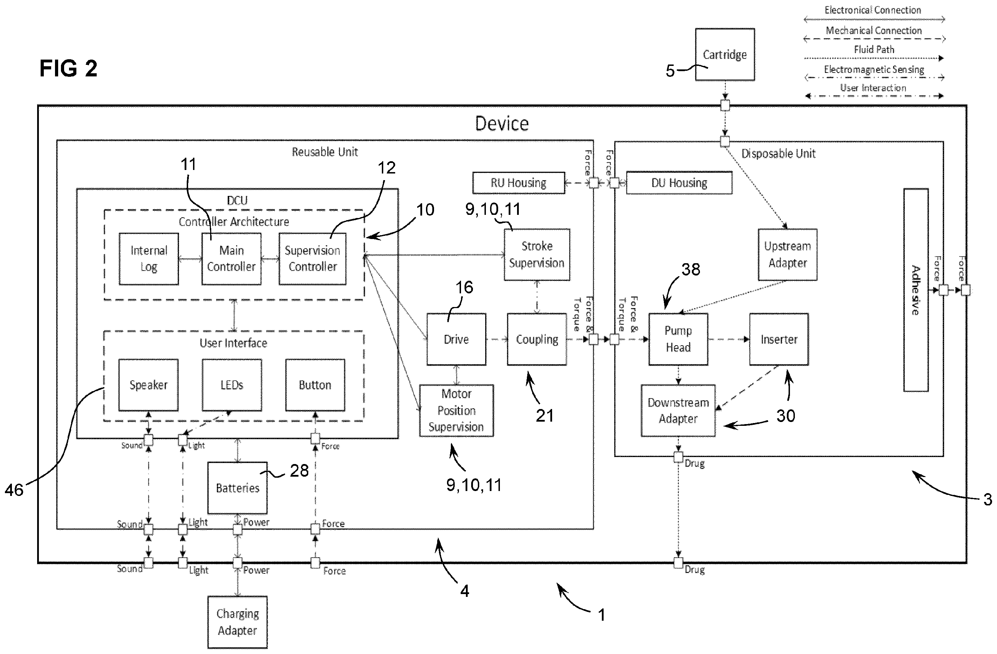

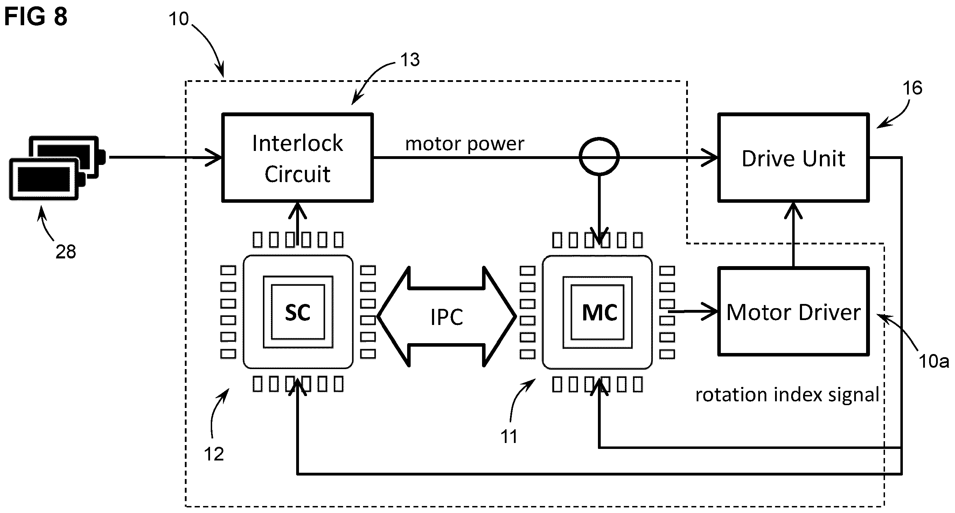

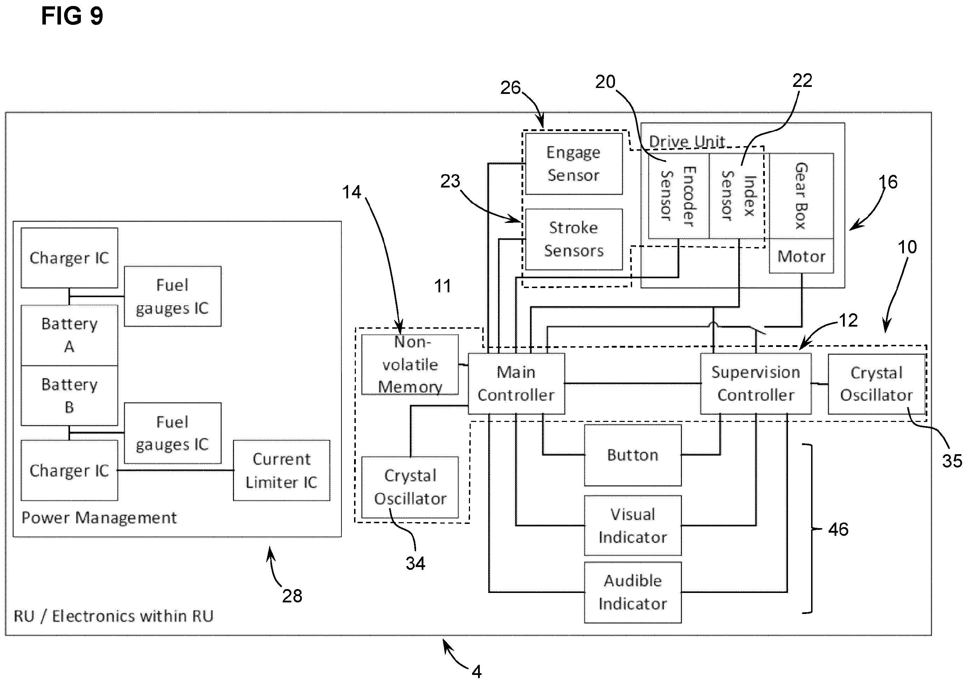

Referring in particular to figures 8, 9 and 2, according to an aspect of the invention, the electronic control system 6 comprises a motor drive circuit 10 including a motor driver controlling operation of the motor 16, the motor driver being controlled by a main controller 11 that receives at least one rotor position sensing signal from the rotor positioning sensing system 9 measuring the rotation of the output transmission shaft 17 of the motor 16. The main controller receives at least the measurement information from the rotational index sensor 22, but may further receive sensing measurement from the rotor position measurement system in the motor and/or from the axial position sensors 23. The main controller determines the operation of the motor driver depending on the desired or specified flow rate whereby if the delivery control module of the motor drive circuit 10 comprises a pulse and pause operating mode as previously described, the motor main controller 11 commands the switching on and switching off of the pulsed operation.

The rotor position sensing system 9 is further connected to a supervision controller 12 which is separate from the main controller 11.

Both the supervision controller and main controller may advantageously be in the form of integrated circuits, separately mounted on the main circuit board 27 and communicating to each other via an Inter-process communication (IPC) protocol. The supervision controller and main controller each have their own clock (crystal oscillator) to ensure completely independent operation.

The supervision controller monitors the operation of the motor 16 based on the rotor position sensing system input into the main controller and the supervision controller, and in case the sensing information does not correspond to the desired or programmed flow rate the supervision controller stops sending toggling signals to an interlock circuit 13 disposed between the power source 28 and the motor 16. The power supply to the motor is supplied through the interlock circuit 13 and in case of a discrepancy between the measured rotor position from the rotor position sensing system 9 and the desired or programmed flow rate, the supervision controller commands the interlock circuit 13 to cut the power to the motor 16. The main controller is configured to control the motor power using a separate circuit which is supplied with power going through the interlock circuit. Both the main and supervision controllers must turn the power on to the motor for motor to operate since the 'interlock circuit' and 'motor driver' are connected in series with the motor.

More specifically, in an embodiment by way of example, the supervision controller receives from the main controller a request to turn on motor power (by a toggling interlock signal) over the IPC interface with the command for motor start, number of motor rotations and time window. The time window is the time within which the next request is expected and depends on the therapy/delivery patterns. For instance there may be two patterns used illustrated by the following example: 17 rotations, delivered every 120 seconds for the first therapy hour (phase 1) and 7 rotations, delivered every 116 seconds for the further four hours of the therapy (phase 2). After reception of the request to turn on motor power, the supervision controller starts three internal software (SW) timers with timeout equal to the received 'time window' extended by 20% (timer 1) and one reduced by 20% (timer 2) and a 3rd timer with timeout equal to the number of rotations multiplied by the maximum time for a motor rotation (timer 3). Afterwards the supervision controller turns on motor power (starts toggling interlock). During the time when timer 3 is running, the supervision controller monitors the number of motor revolutions. If the number of revolutions is higher by more than 1 from the 'number of rotations' requested by the main controller, the supervision controller turns off motor power and sends information about failure to the main controller to raise an alarm. If at the end of timer 3 timeout, the number of counted motor revolutions is smaller than the requested number of rotations by more than one, the supervision controller turns off motor power and sends information about failure to the main controller to raise an alarm. The supervision controller monitors also time elapsed between motor rotation requests. The next motor rotation request is expected between timer 1 and timer 2 expiry. If a new request is received before timer 1 expiry, an over-delivery situation is recognized by the supervision controller. If the next request is not received before timer 2 expiry, then an under-delivery is recognized by the supervision controller. In both cases the supervision controller interrupts motor power and sends an alarm request to the main controller.

The separate main controller and supervision controller thus provide two separate channels for measuring the operation of the motor and thus the pumping operation in an independent manner while at the same time having only one main controller to control the functions, the other serving only to supervise and stop drug delivery in case of a faulty operation being detected.

Action of the supervision controller 12 on the interlock circuit provides a simple and reliable safety mechanism for switching off drug administration in case of faulty operation, in an economical manner. An advantage of using the interlock circuit is that if there is a technical failure of supervision controller leading to permanent logical '1' or '0' on the interlock pin connected to the interlock circuit, the motor power will be disconnected until the interlock circuit receives a permanent changing signal, for instance with frequency about 100Hz, to turn on motor power.

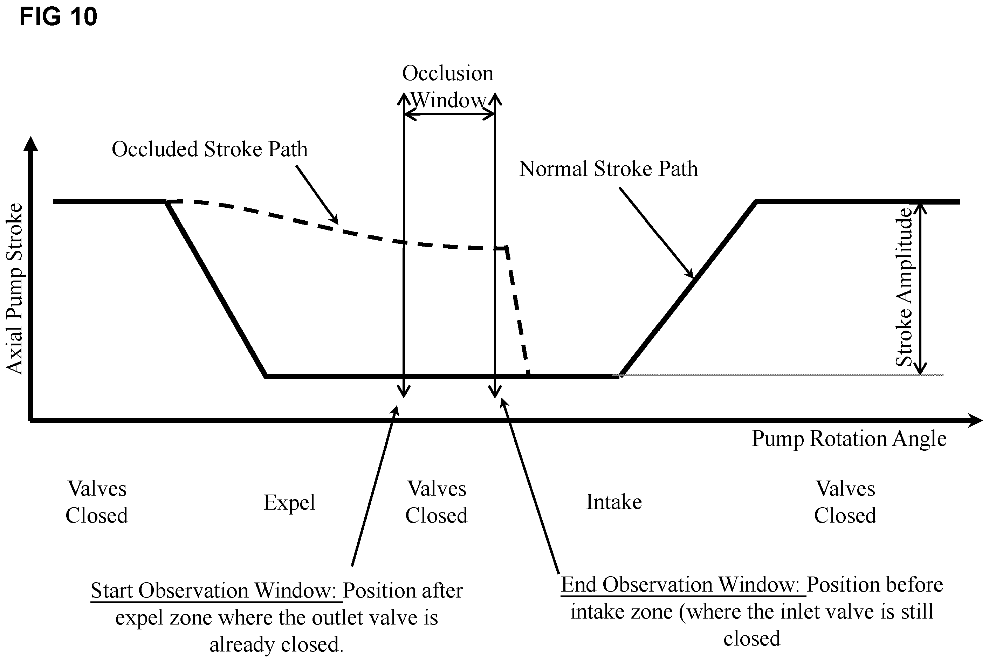

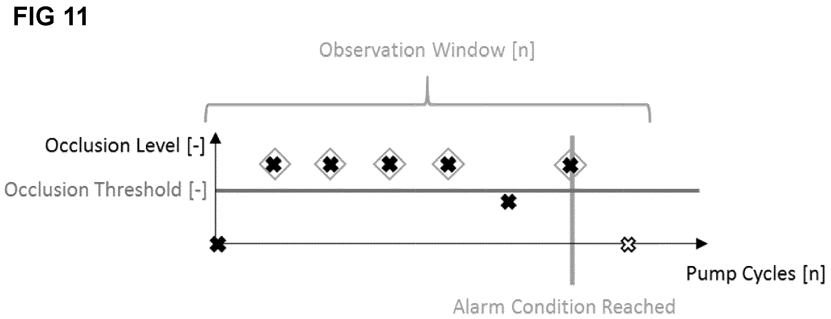

Referring to figures 10 and 11, the delivery supervision software module installed in the motor drive circuit 10 of the electronic control system comprises an occlusion detection module configured to detect occlusion according to the principle of operation illustrated in figure 10. If the fluid flow path is occluded, preventing administration of the drug to the patient, the axial displacement of the pump engine pump rotor 31 is hindered such that the axial pump stroke does not follow the expected path. If the fluid flow downstream of the pump engine is blocked, the pump rotor 31 cannot effect an axial movement since it cannot empty the pump chamber and thus remains at a chamber full or partially full level. An occlusion event may be detected either by measuring an axial position of the rotor, the occlusion event detected if the axial position is measured as a value above an occlusion position threshold, or by measuring an axial displacement amplitude of the rotor, the occlusion event detected if the axial displacement is measured below an occlusion displacement threshold.

As indicated by the dotted line in figure 10 showing the occluded stroke path, each pump cycle (360° rotation of the pump rotor 31) displaces only a small amount of liquid, for instance in a range of 1µL to 10µL, and it may occur that an occluded stroke path is detected from time to time during drug administration due to very short duration temporary blockages. Such temporary blockages may for instance arise due to a temporary movement of the catheter or canula relative to the subcutaneous tissue when the patient makes a movement, or due to bending of the canula or pinching of the catheter tube, or due to a shock on the drug delivery device that disturbs the axial position measurement, or due to some external factor.

If occlusion is detected for one or two cycles due to a temporary event as previously mentioned, this should not set off an alarm since the average flow rate is hardly disturbed and remains within accepted tolerances. According to an aspect of the invention, the occlusion detection module thus requires a minimum of two rotation cycles with an observation window in a range of two to ten successively measured occlusion events, in order to trigger an occlusion detection alarm. Preferably, the observation window is of at least five, for instance between five and eight, whereby if occlusion detection occurs at least three to five times within the observation window of five to eight cycles, the alarm condition is met.

As best seen in figure 10, the axial position of the pump rotor is preferably measured within a certain rotation angle of the rotor that defines an occlusion measurement window within which the measurement is taken at each successive pump cycle (a cycle corresponding to a complete 360° rotation of the pump rotor). This occlusion window preferably is between the end of the expel and the beginning of the intake phases, preferably when both the inlet and outlet valves are closed, this occlusion window being substantially centrally positioned between the end of the expel and the beginning of the intake phases of the pump. The latter ensures that a specific window for detection of the axial position is consistently used at each rotation cycle and that this window is during a valve's closed position for the most reliable position measurement.

The delivery supervision software module and delivery control software module installed in the motor drive circuit 10 of the electronic control system 6 may perform various control and safety functions that are exemplified in the table below.

| **Delivery Supervision Feature** | **Description** | **Implementation** |
|---|---|---|
| **Prevention of unwanted drug delivery** | Confirms that the system is in the right system mode (delivery ready) and a therapy cycle starts upon a long control button press. | The MC and SC run a periodic self-test (PST) |
| | | • Timeout in state machines |
| | Additionally, when responding to a command from the master controller (MC), the supervision controller (SC) will only enable the drive unit by the interlock signal (initiating delivery) when no internal technical error on the SC is pending. | • Check therapy event in a state (state machine) |
| | | • Integrity check of the state of state machine |
| **Detection of erroneous pump rotations** | The SC software checks whether the actual number of pump rotations matches the target number of rotations with a tolerance of 1 rotation. If this check fails, the device enters the Alarm Mode (internal technical error) and safe state (stops delivery, issues alarm, retracts cannula). | The motor driver is controlled by the MC software only. |
| | | A magnetic sensor allows the MC and the SC to measure pump rotation speed, number of rotations and direction. |
| | | The MC and SC run PST |
| **Detection of inconsistency of drug delivery compared with factory-programmed delivery pattern** | The SC software checks whether the drug delivery is performed according to the drug delivery pattern. If this check fails, the device enters the Alarm Mode (internal technical error) and safe state (stops delivery, issues alarm, retracts cannula). | The motor driver is controlled by the MC only. |
| | | A magnetic sensor allows the MC and the SC to measure pump rotation speed, number of rotations and direction. |
| | | The MC and SC run PST |
| **Detection of downstream occlusion** | If the occlusion detection algorithm detects a complete downstream occlusion, the device enters the Alarm Mode and safe state. | The Hall sensors allows the MC to measure pump stroke. |
| | | Collected stroke data from each pump cycle (rotation) are analyzed and supervised by the software via the occlusion detection algorithm. |

### List of references

Drug delivery device 1
**Housing 2**
   Skin contact wall 48
   Adhesive layer 51
   Protective film 50
**Delivery unit 3**
   **Drug container 5**
   Subcutaneous delivery system
      Injection needle / canula
      Needle inserter mechanism
   **Pumping system 8**
   Pump engine 38
      Stator 29
      Pump rotor 31
         Drive coupling interface 40
**Drive unit 4**
   **User interface 46**
      Speaker
      Light(s)
      Activation button
   **Electronic control system 6**
      **Motor drive circuit 10**
         Delivery control module
         Flow rate control module
         Delivery supervision module
            Occlusion detection module
      Main circuit board 27
      Main controller 11
         Integrated circuit
         Crystal oscillator 34
      Supervision controller 12
         Integrated circuit
         Crystal oscillator 35
      Interlock circuit 13
      Memory 14
         Events log
   **Power source 28**
      battery
   **Pump drive 7**
      Motor 16
      Motor output transmission shaft 17
         Coupling side interface
         Slide protuberance(s) / groove(s) 19a
      Spring 36
      Coupling 21
         Pump side interface 39
         Motor side interface 33
            Slide groove(s) / protuberance(s) 19b
      **Rotor position sensing system 9**
         Rotary position sensor
         Rotational index sensor 22
         Axial position sensors (stroke sensor) 23
            Axial position magnet 23a
            First axial position sensor 23b (e.g. Hall sensor)
            Second axial position sensor 23c (e.g. Hall sensor)
               Second circuit board 24
               Holder 25
         Coupling engage sensor 26

## Claims

1. **A drug delivery device** comprising a delivery unit (3) and a drive unit (4), the delivery unit comprising a drug container (5) and a pumping system (8) having a pump engine (38) with a stator (29) and a pump rotor (31) configured to effect an axial piston stroke as a function of the rotation about an axis of rotation of the pump rotor relative to the stator for pumping liquid out of the drug container, the drive unit comprising a housing (2), an electronic control system, a pump drive (7) including a motor (16), a motor output transmission shaft (17) driven in rotation by the motor (16), a rotor position sensing system (9), and a coupling (21) slidably coupled to the motor output transmission shaft (17) driven in rotation by the motor output transmission shaft, the coupling (21) having a pump side interface (39) engaged with a drive coupling interface (40) of the pump rotor (31) configured for transmitting torque from the motor (16) to the pump rotor (31) and for effecting an axial displacement following an axial displacement of the pump rotor (31), **wherein** the electronic control system (6) comprises a motor drive circuit (10) connected to the motor (16) of the pump drive (7) and at least one rotary position sensor of the rotor position sensing system (9) for controlling the motor and thereby the pumping of a drug from the drug container (5) with the pumping system (8), the motor drive circuit (10) including a main controller (11) controlling signals for operation of the motor, **characterised in that** the motor drive circuit (10) further includes a supervision controller (12) separate from the main controller (11) and also connected to said at least one rotary position sensor of the rotor position sensing system (9), the supervision controller (12) connected to an interlock circuit configured to transmit electrical power from a power source (28) to the motor (16), the supervision controller (12) configured to actuate the interlock circuit to interrupt power to the motor (16) if the rotor position measurement signal received from the at least one rotary position sensor does not match a set-point value within a predefined tolerance threshold.

2. The drug delivery device according to the preceding claim wherein the rotor position measurement signal comprises or consists of a number of rotations of the rotor and the predefined tolerance threshold is one or more rotations above or below the set-point value.

3. The drug delivery device according to the preceding claim wherein the main controller (11) comprises or consists of an integrated circuit, and the supervision controller (12) comprises or consists of an integrated circuit, the supervision controller and main controller being supplied with power and sensing signals independently of each other.

4. The drug delivery device according to any preceding claim wherein the main controller and the supervision controller each comprise their own crystal oscillator.

5. The drug delivery device according to any preceding claim wherein the motor drive circuit comprises a delivery control module, the delivery control module receiving an axial position measurement of the pump rotor (31) as a function of the rotational position of the pump rotor, wherein the delivery control module is configured to emit an alarm condition defining an occlusion event of the drug delivery device triggered after an axial position of the rotor is measured as a value above an occlusion position threshold, or after an axial displacement amplitude of the rotor is measured below an occlusion displacement threshold, for at least two revolutions of the pump rotor (31) within a window of at least three to no more than ten revolutions.

6. The drug delivery device according to the preceding claim wherein said window is in a range of 5 to 8 revolutions and said alarm condition defining an occlusion event of the drug delivery device is triggered after an axial position of the rotor is measured as a value above an occlusion position threshold, or after an axial displacement amplitude of the rotor is measured below an occlusion displacement threshold, for at least three revolutions of the pump rotor.

7. The drug delivery device according to either of the two directly preceding claims wherein the rotor axial position used for determining if the occlusion position threshold has been passed, or the rotor axial displacement used for determining if the occlusion displacement threshold has been passed, is measured within an occlusion window defined by a rotational position of the rotor between an end of expel and a beginning of intake of a pump cycle defined by one revolution of the rotor, the occlusion window being in a both valves closed angular range.

8. The drug delivery device according to any preceding claim wherein the motor drive circuit comprises a delivery control module for controlling operation of the motor (16) of the pump drive (7), the delivery control module configured to operate the motor in a plurality of pulse and pause sequencies, the pulse and pause times adjusted to achieve a specified average flow rate, each pulse operation comprising a plurality of motor output transmission shaft revolutions, the average flow rate being adjusted by varying the number of revolutions per pulse, or the duration of the pause between pulses.

9. The drug delivery device according to the preceding claim wherein the rotor position sensing system (9) is connected to the electronic control system (6) configured to supply at least a rotor position measurement at least once per revolution to the delivery control module wherein the output transmission shaft is configured to be stopped by the delivery control module in a predefined park position at the end of each pulse operation.

10. The drug delivery device according to either of the two directly preceding claims wherein the delivery control module is configured to operate pulses having at least three revolutions per pulse, an average flow rate being adjusted by the pause duration alone, or in combination with the pulse duration.

11. The drug delivery device according to any of the three directly preceding claims wherein the electronic control system is configured to operate a pulse and pause by switching the motor on and off, a voltage and current supply to the motor supplied configured for operation of the motor at a preset optimal power.

12. The drug delivery device according to any preceding claim wherein the rotor position sensing system (9) includes at least one rotary position sensor (20, 22) measuring a rotation of the motor output transmission shaft, and an axial sensor (23) for measuring an axial displacement of the pump rotor, the axial sensor (23) comprising a first axial position sensor (23b) mounted statically in the housing of the drive unit, and a second axial position sensor (23c) mounted statically in the housing of the drive unit, the first and second axial position sensors arranged on opposite sides of the rotation axis, the first and second angular positions being separated by 180°.

13. The drug delivery device according to the preceding claim wherein the electronic control system (6) is configured to measure an axial position of the coupling side interface (18) by processing a sum or an average value of the measurement outputs of the first and second axial position sensors (23b, 23c).

14. The drug delivery device according to either of the two directly preceding claims wherein the axial position sensor (23) comprises an axial position magnet (23a) mounted on the coupling (21) with an annular portion that encircles the rotation axis of the coupling, the first and second axial position sensors comprising first and second magnetic field sensors (23b, 23c).

15. The drug delivery device according to any of the three directly preceding claims wherein the rotary position sensor (20) comprises an integrated incremental solver in the motor.

## Patentansprüche

1. Arzneimittelabgabevorrichtung, die eine Abgabeeinheit (3) und eine Antriebseinheit (4) umfasst, wobei die Abgabeeinheit einen Arzneimittelbehälter (5) und ein Pumpsystem (8) umfasst, das einen Pumpenmotor (38) mit einem Stator (29) und einen Pumpenrotor (31) aufweist, der dazu ausgestaltet ist, einen axialen Kolbenhub als eine Funktion der Drehung um eine Drehachse des Pumpenrotors in Bezug auf den Stator zum Pumpen von Flüssigkeit aus dem Arzneimittelbehälter durchzuführen, wobei die Antriebseinheit ein Gehäuse (2), ein elektronisches Steuerungssystem, einen Pumpenantrieb (7), der einen Motor (16) umfasst, eine Motorabtriebsübertragungswelle (17), die von dem Motor (16) drehbar angetrieben wird, ein RotorpositionsErfassungssystem (9) und eine Kopplung (21) umfasst, die verschiebbar an die Motorabtriebsübertragungswelle (17) gekoppelt ist und von der Motorabtriebsübertragungswelle drehbar angetrieben wird, wobei die Kopplung (21) eine pumpenseitige Grenzfläche (39) aufweist, die mit einer Antriebskopplungsgrenzfläche (40) des Pumpenrotors (31) in Eingriff steht, die dazu ausgestaltet ist, Moment von dem Motor (16) auf den Pumpenrotor (31) zu übertragen und eine axiale Verlagerung durchzuführen, die einer axialen Verlagerung des Pumpenrotors (31) folgt, wobei das elektronische Steuersystem (6) eine Motorantriebsschaltung (10), die mit den Motor (16) des Pumpenantriebs (7) verbunden ist, und mindestens einen Drehpositionssensor des Rotorpositionserfassungssystems (9) zum Steuern des Motors und dadurch des Pumpens eines Arzneimittels von dem Arzneimittelbehälter (5) mit dem Pumpsystem (8) umfasst, wobei die Motorantriebsschaltung (10) eine Hauptsteuereinrichtung (11) umfasst, die Signale zum Betrieb des Motors steuert, **dadurch gekennzeichnet, dass** die Motorantriebsschaltung (10) ferner eine Überwachungssteuereinrichtung (12) getrennt von der Hauptsteuereinrichtung (11) umfasst und auch mit dem mindestens einen Drehpositionssensor des Rotorpositionserfassungssystems (9) verbunden ist, wobei die Überwachungssteuereinrichtung (12) mit einer Verriegelungsschaltung verbunden ist, die dazu ausgestaltet ist, elektrische Leistung von einer Leistungsquelle (28) auf den Motor (16) zu übertragen, wobei die Überwachungssteuereinrichtung (12) dazu ausgestaltet ist, die Verriegelungsschaltung zu betätigen, um Leistung für den Motor (16) zu unterbrechen, wenn das Rotorpositionsmesssignal, das von dem mindestens einen Drehpositionssensor empfangen wird, nicht mit einem Sollwert innerhalb eines vorbestimmten Toleranzschwellenwerts übereinstimmt.

2. Arzneimittelabgabevorrichtung nach dem vorhergehenden Anspruch, wobei das Rotorpositionsmesssignal eine Anzahl von Umdrehungen des Rotors umfasst oder daraus besteht und der vordefinierte Toleranzschwellenwert eine oder mehrere Umdrehungen über oder unter dem Sollwert liegt.

3. Arzneimittelabgabevorrichtung nach dem vorhergehenden Anspruch, wobei die Hauptsteuereinrichtung (11) eine integrierte Schaltung umfasst oder daraus besteht und die Überwachungssteuereinrichtung (12) eine integrierte Schaltung umfasst oder daraus besteht, wobei die Überwachungssteuereinrichtung und die Hauptsteuereinrichtung unabhängig voneinander mit Leistung und Erfassungssignalen versorgt werden.

4. Arzneimittelabgabevorrichtung nach einem der vorhergehenden Ansprüche, wobei die Hauptsteuereinrichtung und die Überwachungssteuereinrichtung jeweils ihren eigenen Quarzoszillator umfassen.

5. Arzneimittelabgabevorrichtung nach einem der vorhergehenden Ansprüche, wobei die Motorantriebsschaltung ein Abgabesteuerungsmodul umfasst, wobei das Abgabesteuerungsmodul eine axiale Positionsmessung des Pumpenrotors (31) als eine Funktion der Drehposition des Pumpenrotors empfängt, wobei das Abgabesteuerungsmodul dazu ausgestaltet ist, einen Alarmzustand auszugeben, der ein Verstopfungsereignis der Arzneimittelabgabevorrichtung definiert und ausgelöst wird, nachdem eine axiale Position des Rotors als ein Wert über einem Verstopfungspositionsschwellenwert gemessen wurde oder nachdem eine axiale Verlagerungsamplitude des Rotors während mindestens zwei Umdrehungen des Pumpenrotors (31) innerhalb eines Fensters von mindestens drei bis nicht mehr als zehn Umdrehungen unter einem Verstopfungsverlagerungsschwellenwert gemessen wurde.

6. Arzneimittelabgabevorrichtung nach dem vorhergehenden Anspruch, wobei das Fenster in einem Bereich von 5 bis 8 Umdrehungen liegt und der Alarmzustand, der ein Verstopfungsereignis der Arzneimittelabgabevorrichtung definiert, ausgelöst wird, nachdem eine axiale Position des Rotors als ein Wert über einem Verstopfungspositionsschwellenwert gemessen wurde oder nachdem eine axiale Verlagerungsamplitude des Rotors während mindestens drei Umdrehungen des Pumpenrotors unter einem Verstopfungsverlagerungsschwellenwert gemessen wurde.

7. Arzneimittelabgabevorrichtung nach einem der zwei direkt vorhergehenden Ansprüche, wobei die axiale Rotorposition, die verwendet wird, um zu bestimmen, ob der Verstopfungspositionsschwellenwert überschritten wurde, oder die axiale Rotorverlagerung, die verwendet wird, um zu bestimmen, ob der Verstopfungsverlagerungsschwellenwert überschritten wurde, innerhalb eines Verstopfungsfensters gemessen wird, das durch eine Drehposition des Rotors zwischen einem Ende eines Ausstoßes und einem Anfang eines Einlasses eines Pumpenzyklus definiert ist, der durch eine Umdrehung des Rotors definiert ist, wobei das Verstopfungsfenster in einem Winkelbereich mit beiden geschlossenen Ventilen liegt.

8. Arzneimittelabgabevorrichtung nach einem der vorhergehenden Ansprüche, wobei die Motorantriebsschaltung ein Abgabesteuerungsmodul zum Steuern des Betriebs des Motors (16) des Pumpenantriebs (7) umfasst, wobei das Abgabesteuerungsmodul dazu ausgestaltet ist, den Motor in einer Vielzahl von Impuls- und Pausensequenzen zu betreiben, wobei die Impuls- und Pausenzeiten angepasst werden, um eine angegebene durchschnittliche Strömungsgeschwindigkeit zu erreichen, wobei jeder Impulsbetrieb eine Vielzahl von Motorabgabeübertragungswellenumdrehungen umfasst, wobei die durchschnittliche Strömungsgeschwindigkeit durch Variieren der Anzahl von Umdrehungen pro Impuls oder der Dauer der Pause zwischen Impulsen angepasst wird.

9. Arzneimittelabgabevorrichtung nach dem vorhergehenden Anspruch, wobei das Rotorpositionserfassungssystem (9) mit dem elektronischen Steuersystem (6) verbunden ist, das dazu ausgestaltet ist, mindestens eine Rotorpositionsmessung mindestens ein Mal pro Umdrehung an das Abgabesteuerungsmodul zu liefern, wobei die Abgabeübertragungswelle dazu ausgestaltet ist, am Ende jedes Impulsbetriebs durch das Abgabesteuerungsmodul in einer vordefinierten Parkposition angehalten zu werden.

10. Arzneimittelabgabevorrichtung nach einem der zwei direkt vorhergehenden Ansprüche, wobei das Abgabesteuerungsmodul dazu ausgestaltet ist, Impulse zu betreiben, die mindestens drei Umdrehungen pro Impuls aufweisen, wobei eine durchschnittliche Strömungsgeschwindigkeit einzig mittels der Pausendauer oder in Kombination mit der Impulsdauer angepasst wird.

11. Arzneimittelabgabevorrichtung nach einem der drei direkt vorhergehenden Ansprüche, wobei das elektronische Steuerungssystem dazu ausgestaltet ist, einen Impuls und eine Pause mittels Ein- und Ausschaltens des Motors zu betreiben, wobei eine Spannungs- und Stromversorgung für den versorgten Motor für den Betrieb des Motors bei einer voreingestellten optimalen Leistung ausgestaltet ist.

12. Arzneimittelabgabevorrichtung nach einem der vorhergehenden Ansprüche, wobei das Rotorpositionserfassungssystem (9) mindestens einen Drehpositionssensor (20, 22), der eine Umdrehung der Motorabgabeübertragungswelle misst, und einen axialen Sensor (23) zum Messen einer axialen Verlagerung des Pumpenrotors umfasst, wobei der axiale Sensor (23) einen ersten Axialpositionssensor (23b) umfasst, der statisch im Gehäuse der Antriebseinheit montiert ist, und einen zweiten Axialpositionssensor (23c), umfasst, der statisch im Gehäuse der Antriebseinheit montiert ist, wobei der erste und der zweite Axialpositionssensor auf entgegengesetzten Seiten der Drehachse angeordnet sind, wobei die erste und die zweite Winkelposition um 180° getrennt sind.

13. Arzneimittelabgabevorrichtung nach dem vorhergehenden Anspruch, wobei das elektronische Steuerungssystem (6) dazu ausgestaltet ist, eine axiale Position der kopplungsseitigen Grenzfläche (18) mittels Verarbeitens einer Summe oder eines Durchschnittswerts der Messausgänge des ersten und des zweiten Axialpositionssensors (23b, 23c) zu messen.

14. Arzneimittelabgabevorrichtung nach einem der zwei direkt vorhergehenden Ansprüche, wobei der Axialpositionssensor (23) einen Axialpositionsmagneten (23a) umfasst, der an der Kopplung (21) mit einem ringförmigen Abschnitt montiert ist, der die Drehachse der Kopplung umgibt, wobei der erste und der zweite Axialpositionssensor einen ersten und einen zweiten Magnetfeldsensor (23b, 23c) umfassen.

15. Arzneimittelabgabevorrichtung nach einem der drei direkt vorhergehenden Ansprüche, wobei der Drehpositionssensor (20) einen integrierten inkrementellen Solver im Motor umfasst.

## Revendications

1. Dispositif de distribution de médicament comprenant une unité de distribution (3) et une unité d'entraînement (4), l'unité de distribution comprenant un récipient de médicament (5) et un système de pompage (8) ayant un moteur de pompe (38) avec un stator (29) et un rotor de pompe (31) configuré pour effectuer une course de piston axiale en fonction de la rotation autour d'un axe de rotation du rotor de pompe par rapport au stator pour pomper le liquide hors du récipient de médicament, l'unité d'entraînement comprenant un boîtier (2), un système électronique de commande, un entraînement de pompe (7) comprenant un moteur (16), un arbre de transmission de sortie de moteur (17) entraîné en rotation par le moteur (16), un système de détection de position de rotor (9), et un couplage (21) couplé, de manière coulissante, à l'arbre de transmission de sortie de moteur (17) entraîné en rotation par l'arbre de transmission de sortie de moteur, le couplage (21) ayant une interface du côté de la pompe (39) mise en prise avec une interface de couplage d'entraînement (40) du rotor de pompe (31) configurée pour transmettre le couple du moteur (16) au rotor de pompe (31) et pour effectuer un déplacement axial suite à un déplacement axial du rotor de pompe (31), dans lequel le système électronique de commande (6) comprend un circuit d'entraînement de moteur (10) raccordé au moteur (16) de l'entraînement de pompe (7) et au moins un capteur de position rotatif du système de détection de position de rotor (9) pour commander le moteur et ainsi le pompage d'un médicament du récipient de médicament (5) avec le système de pompage (8), le circuit d'entraînement de moteur (10) comprend un contrôleur principal (11) commandant les signaux pour le fonctionnement du moteur, **caractérisé en ce que** le circuit d'entraînement de moteur (10) comprend en outre un contrôleur de supervision (12) séparé du contrôleur principal (11) et également raccordé audit au moins un capteur de position rotatif du système de détection de position de rotor (9), le contrôleur de supervision (12) étant raccordé à un circuit de sécurité configuré pour transmettre l'énergie électrique d'une source d'alimentation (28) au moteur (16), le contrôleur de supervision (12) étant configuré pour actionner le circuit de sécurité afin d'interrompre l'arrivée de l'énergie au moteur (16), si le signal de mesure de position de rotor reçu du au moins un capteur de position rotatif ne correspond pas à une valeur de consigne dans un seuil de tolérance prédéfini.

2. Dispositif de distribution de médicament selon la revendication précédente, dans lequel le signal de mesure de position de rotor comprend ou se compose d'un certain nombre de rotations du rotor et le seuil de tolérance prédéfini est une ou plusieurs rotations au-dessus ou au-dessous de la valeur de consigne.

3. Dispositif de distribution de médicament selon la revendication précédente, dans lequel le contrôleur principal (11) comprend ou se compose d'un circuit intégré, et le contrôleur de supervision (12) comprend ou se compose d'un circuit intégré, le contrôleur de supervision et le contrôleur principal peuvent être alimentés avec l'énergie et les signaux de détection indépendamment l'un de l'autre.

4. Dispositif de distribution de médicament selon l'une quelconque des revendications précédentes, dans lequel le contrôleur principal et le contrôleur de supervision comprennent chacun leur propre oscillateur à quartz.

5. Dispositif de distribution de médicament selon l'une quelconque des revendications précédentes, dans lequel le circuit d'entraînement de moteur comprend un module de commande de distribution, le module de commande de distribution recevant une mesure de position axiale du rotor de pompe (31) en fonction de la position de rotation du rotor de pompe, dans lequel le module de commande de distribution est configuré pour émettre une condition d'alarme définissant un évènement d'occlusion du dispositif de distribution de médicament déclenchée après qu'une position axiale du rotor a été mesurée en tant que valeur supérieure à un seuil de position d'occlusion, ou après qu'une amplitude de déplacement axial du rotor a été mesurée au-dessous d'un seuil de déplacement d'occlusion, pendant au moins deux révolutions du rotor de pompe (31) dans une fenêtre d'au moins trois jusqu'à une valeur non supérieure à dix révolutions.

6. Dispositif de distribution de médicament selon la revendication précédente, dans lequel ladite fenêtre est dans la plage de 5 à 8 révolutions et ladite condition d'alarme définissant un évènement d'occlusion du dispositif de distribution de médicament est déclenchée après qu'une position axiale du rotor a été mesurée en tant que valeur supérieure à un seuil de position d'occlusion, ou après qu'une amplitude de déplacement axial du rotor a été mesurée au-dessous d'un seuil de déplacement d'occlusion, pendant au moins trois révolutions du rotor de pompe.

7. Dispositif de distribution de médicament selon l'une ou l'autre des deux revendications directement précédentes, dans lequel la position axiale de rotor utilisée pour déterminer si le seuil de position d'occlusion est passé, ou le déplacement axial de rotor utilisé pour déterminer si le seuil de déplacement d'occlusion est passé, est mesuré(e) dans une fenêtre d'occlusion définie par une position de rotation du rotor entre une fin d'expulsion et un début d'admission d'un cycle de pompe défini par une révolution du rotor, la fenêtre d'occlusion étant dans une plage angulaire où les deux valves sont fermées.

8. Dispositif de distribution de médicament selon l'une quelconque des revendications précédentes, dans lequel le circuit d'entraînement de moteur comprend un module de commande de distribution pour commander le fonctionnement du moteur (16) de l'entraînement de pompe (7), le module de commande de distribution étant configuré pour faire fonctionner le moteur dans une pluralité de séquences à impulsion et pause, les temps d'impulsion et de pause étant ajustés pour atteindre un débit moyen spécifié, chaque opération d'impulsion comprenant une pluralité de révolutions d'arbre de transmission de sortie de moteur, le débit moyen étant ajusté en modifiant le nombre de révolutions par impulsion ou la durée de la pause entre les impulsions.

9. Dispositif de distribution de médicament selon la revendication précédente, dans lequel le système de détection de position de rotor (9) est raccordé au système électronique de commande (6) configuré pour fournir au moins une mesure de position de rotor, au moins une fois par révolution, au module de commande de distribution, dans lequel l'arbre de transmission de sortie est configuré pour être arrêté par le module de commande de distribution dans une position de repos à la fin de chaque opération d'impulsion.

10. Dispositif de distribution de médicament selon l'une ou l'autre des deux revendications directement précédentes, dans lequel le module de commande de distribution est configuré pour actionner les impulsions ayant au moins trois révolutions par impulsion, un débit moyen étant ajusté par la durée de pause seule, ou en combinaison avec la durée d'impulsion.

11. Dispositif de distribution de médicament selon l'une quelconque des trois revendications directement précédentes, dans lequel le système électronique de commande est configuré pour actionner une impulsion et une pause en commutant la marche et l'arrêt du moteur, une alimentation en tension et en courant par rapport au moteur configurée pour faire fonctionner le moteur à une puissance optimale prédéfinie.

12. Dispositif de distribution de médicament selon l'une quelconque des revendications précédentes, dans lequel le système de détection de position de rotor (9) comprend au moins un capteur de position rotatif (20, 22) mesurant une rotation de l'arbre de transmission de sortie de moteur, et un capteur axial (23) pour mesurer un déplacement axial du rotor de pompe, le capteur axial (23) comprenant un premier capteur de position axiale (23b) monté de manière statique dans le boîtier de l'unité d'entraînement, et un second capteur de position axiale (23c) monté de manière statique dans le boîtier de l'unité d'entraînement, les premier et second capteurs de position axiale étant agencés sur les côtés opposés de l'axe de rotation, les première et seconde positions angulaires étant séparées de 180°.

13. Dispositif de distribution de médicament selon la revendication précédente, dans lequel le système électronique de commande (6) est configuré pour mesurer une position axiale de l'interface du côté du couplage (18) en traitant une somme ou une valeur moyenne des résultats de mesure des premier et second capteurs de position axiale (23b, 23c).

14. Dispositif de distribution de médicament selon l'une ou l'autre de deux revendications directement précédentes, dans lequel le capteur de position axiale (23) comprend un aimant de position axiale (23a) monté sur le couplage (21) avec une partie annulaire qui encercle l'axe de rotation du couplage, les premier et second capteurs de position axiale comprennent des premier et second capteurs de champ magnétique (23b, 23c).

15. Dispositif de distribution de médicament selon l'une quelconque des trois revendications directement précédentes, dans lequel le capteur de position rotatif (20) comprend un module de résolution d'incréments intégré dans le moteur.
